# EUROPEAN PATENT APPLICATION

(11) **EP 2 583 686 A1**
(43) Date of publication of application: **24.04.2013**
(21) Application number: 11795597.1
(22) Date of filing: 07.06.2011
(51) Int. Cl.: A61K 38/43, A61K 35/74, A61K 36/06, A61P 1/02, A61P 35/00, A61P 43/00

(54) **AGENT FOR REDUCING ACETALDEHYDE IN ORAL CAVITY**

(30) Priority: 19.06.2010 JP 2010140026
(71) Applicant: Amano Enzyme Inc., Nagoya-shi Aichi 460-8630 (JP)
(72) Inventor: YAMASHIRO Kan, Kakamigahara-shi Gifu 509-0109 (JP); KOYAMA Takahumi, Kakamigahara-shi Gifu 509-0109 (JP)
(74) Representative: Steinecke, Peter
(86) International application number: PCT/JP2011/062991
(87) International publication number: WO 2011/158689

(57) **Abstract**

Disclosed herein is a novel enzymatic agent effective in reducing acetaldehyde in the oral cavity. It has been found that an aldehyde dehydrogenase derived from a microorganism belonging to the genus Saccharomyces and a threonine aldolase derived from Escherichia coli are effective in reducing low concentrations of acetaldehyde. Therefore, an agent for reducing acetaldehyde in the oral cavity is provided, which contains these enzymes as active ingredients.

## Description

### TECHNICAL FIELD

The present invention relates to an agent for reducing acetaldehyde in the oral cavity. More specifically, the present invention relates to an agent that reduces acetaldehyde in the oral cavity by an acetaldehyde-degrading enzyme and a use thereof. This application is based upon and claims the benefit of priority from prior Japanese Patent Application No. 2010-140026 filed on June 19, 2010 in Japan, the entire contents of which are incorporated herein by reference.

### BACKGROUND ART

In recent years, it has been reported that acetaldehyde in saliva not only causes bad breath but also increases the risk of diseases such as upper digestive system cancers. Acetaldehyde in saliva is derived from various sources, but in recent years, acetaldehyde resulting from alcohol metabolism during alcohol drinking or from smoking or acetaldehyde derived from periodontal pathogens in the mouth has been known as a typical example and regarded as a problem.

Among them, acetaldehyde produced as a metabolic product in the course of alcoholysis accumulates in saliva during alcohol drinking, which constitutes a risk factor for esophageal cancer and stomach cancer. Particularly, it has been reported that in the case of flushers (people whose genome type of aldehyde dehydrogenase is AG or GG), the concentration of acetaldehyde in saliva is increased up to almost 100 µM by alcohol drinking.

It is also known that the concentration of acetaldehyde in saliva is increased by smoking. It is considered that one of its causes is acetaldehyde contained in tobacco smoke (Filtration of tobacco smoke (written by Otani Y), The history of smoking research: 1996-2005, edited by Misu Y, Agari I, Ohwada H, Nakao K, and Itani S, Smoking Research Foundation, 2007).

A research group of Tokyo University has reported that drinking of one or more cans of beer per day and smoking makes the risk of esophageal cancer for flushers up to 190 times higher than that for people who are not flushers and do not drink alcohol and smoke (Cui R, Kamatani Y, et. al. Functional variants in ADH1B and ALDH2 coupled with alcohol and smoking synergistically enhance esophageal cancer risk. Gastroenterology 137:1768-1775 (2009)).

It has been found by epidemiological studies that some bacteria present in the mouth synthesize acetaldehyde and acetaldehyde derived from such bacteria is a cause of bad breath or a risk factor for upper digestive system cancers such as esophageal cancer and stomach cancer (which was reported at the annual meeting of the Japanese Cancer Association in 2009). According to the results of an epidemiological study performed by Aichi Cancer Center Research Institute (in Nagoya City) on about 3,800 people, people who brush teeth twice or more a day have a lower risk for oral cancer or esophageal cancer by 30% than people who brush teeth once a day. It is considered that the reason for this is that oral bacteria that produce acetaldehyde and produced acetaldehyde are washed away by tooth brushing.

Meanwhile, as means for removing acetaldehyde in saliva or foods, one using L-cysteine (Non-Patent Document 1) and one using an aldehyde oxidase derived from a microorganism (Patent Document 1) are known. The former utilizes the property of L-cysteine to bind to acetaldehyde. However, this reaction is a reversible reaction, and therefore there is a possibility that acetaldehyde is again liberated. Further, L-cysteine itself has a distinctive smell, which makes it difficult to apply L-cysteine to oral care products. On the other hand, the aldehyde oxidase derived from a microorganism is advantageous in that it does not require a coenzyme, but there is a problem that hazardous hydrogen peroxide is generated by a reaction involving the aldehyde oxidase. Further, the reported aldehyde oxidase derived from a microorganism exhibits activity under acidic conditions (at around pH 3.5), but its reactivity under physiological conditions is unknown. Furthermore, the confirmed reactivity of the enzyme is nothing more than reactivity only toward relatively high concentrations (in the order of mM) of substrate. The concentration of acetaldehyde contained in saliva is usually in the order of µM, but the reactivity of an enzymatic reaction is lowered when the concentration of a substrate is Km or less. Therefore, there is a demand for development of an enzyme that has sufficient activity against low concentrations of substrate. It is to be noted that it has been reported that a superoxide dismutase derived from a microorganism belonging to the genus Gluconobacter exhibits acetaldehyde-degrading activity (Patent Document 2).

### PRIOR ART DOCUMENTS

### PATENT DOCUMENTS

Patent Document 1: JP 2010-57482 A
Patent Document 2: JP 2010-110248 A

### Non-Patent Document

Non-Patent Document 1: Salaspuro V, Hietala J, Kaihovaara P, et. al., Int J Cancer. 2002 Jan 20; 97(3):361-4.

### SUMMRY OF THE INVENTION

### PROBLEMS TO BE SOLVED BY THE INVENTION

As has been described above, it has been found that acetaldehyde in the oral cavity (in the mouth) poses a risk for health problems, and therefore there is a demand for development of oral care products that can degrade and remove such acetaldehyde. It is therefore an object of the present invention to provide a novel enzyme preparation effective in reducing acetaldehyde in the oral cavity.

### MEANS FOR SOLVING THE PROBLEMS

The present inventors have intensively studied using artificial saliva to find enzymes effective in reducing acetaldehyde in the oral cavity. As a result, the present inventors have found that an aldehyde dehydrogenase and a threonine aldolase exhibit excellent activity at low substrate concentrations, which has led to the completion of the present invention. The present invention includes the following (1) to (10).
(1) An agent for reducing acetaldehyde in an oral cavity, including an aldehyde dehydrogenase or a threonine aldolase.
(2) The agent for reducing acetaldehyde in an oral cavity according to (1), wherein the aldehyde dehydrogenase is derived from a microorganism belonging to the genus Saccharomyces.
(3) The agent for reducing acetaldehyde in an oral cavity according to (2), wherein the microorganism belonging to the genus Saccharomyces is Saccharomyces cerevisiae.
(4) The agent for reducing acetaldehyde in an oral cavity according to (1), wherein the threonine aldolase is derived from Escherichia coli.
(5) The agent for reducing acetaldehyde in an oral cavity according to any one of (1) to (4), which exhibits degradation activity against acetaldehyde at a substrate concentration of 1 µM to 1 mM.
(6) The agent for reducing acetaldehyde in an oral cavity according to any one of (1) to (4), which exhibits degradation activity against acetaldehyde at a substrate concentration of 1 µM to 100 µM.
(7) An oral composition including the agent for reducing acetaldehyde in an oral cavity according to any one of (1) to (6).
(8) A method for reducing acetaldehyde in saliva by allowing the oral composition according to (7) to act on saliva.
(9) The method according to (8) which is effective in reducing oral odor.
(10) The method according to (8) which is effective in preventing cancer whose cause or one of causes is acetaldehyde.

### BRIEF DESCRIPTION OF THE DRAWINGS

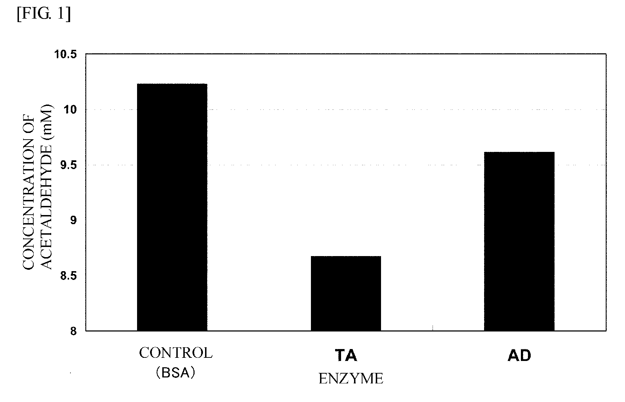

Fig. 1 is a graph showing the concentrations of acetaldehyde in artificial saliva after enzymes were added to evaluate the activity of each of the enzymes by Nash's method, wherein AD, TA, and BSA represent an aldehyde dehydrogenase, a threonine aldolase, and bovine serum albumin, respectively.

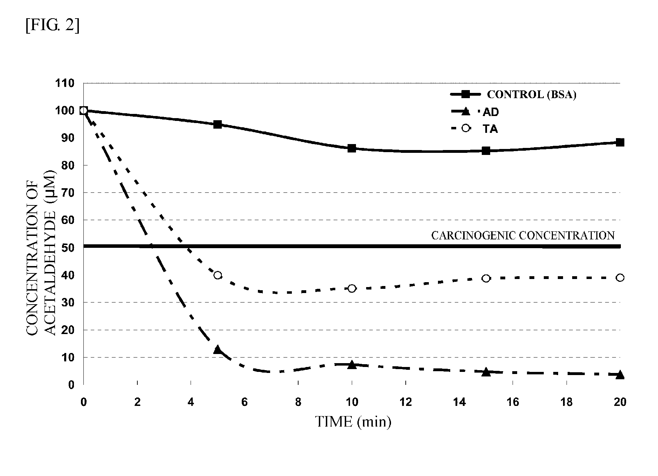

Fig. 2 is a graph showing temporal changes in the concentrations of acetaldehyde in artificial saliva after enzymes were added to evaluate the activity of each of the enzymes by GC-MS, wherein AD, TA, and BSA represent an aldehyde dehydrogenase, a threonine aldolase, and bovine serum albumin, respectively.

### DESCRIPTION OF EMBODIMENTS

In this description, the term "agent for reducing acetaldehyde in the oral cavity" refers to an agent which is intended for use in the oral cavity and the use of which makes it possible to reduce the concentration of acetaldehyde in the oral cavity (more specifically, in saliva). It is to be noted that the term "in the oral cavity" used in this description can be replaced by the term "in the mouth".

### 1. Agent for Reducing Acetaldehyde

A first aspect of the present invention relates to an agent for reducing acetaldehyde in the oral cavity (hereinafter, abbreviated as "acetaldehyde-reducing agent" for convenience of description). The acetaldehyde-reducing agent according to the present invention contains an aldehyde dehydrogenase or a threonine aldolase as an active ingredient. These two enzymes may be used in combination. As the aldehyde dehydrogenase, one derived from a microorganism belonging to the genus Saccharomyces (e.g., Saccharomyces cerevisiae) is preferably used. The aldehyde dehydrogenase derived from Saccharomyces cerevisiae is available from, for example, Sigma-Aldrich, Roche-Diagnostics, or Merk. On the other hand, as the threonine aldolase, one derived from Escherichia coli (see, for example, Eur. J. Biochem, 255, 220-226(1998)) is preferably used.

The concentration of acetaldehyde (substrate) at which the acetaldehyde-reducing agent according to the present invention can act is not particularly limited, but is preferably in the range of 1 µM to 1 mM, more preferably in the range of 1 µM to 100 µM. It is to be noted that the strength (degree) of activity of the acetaldehyde-reducing agent according to the present invention is not particularly limited.

The acetaldehyde-reducing agent according to the present invention may contain, in addition to the active ingredient (the aldehyde dehydrogenase or the threonine aldolase), an excipient, a buffering agent, a suspension agent, a stabilizer, a pH adjuster, a preservative, an antiseptic, a perfume, a thickener, fat and oil, a gloss agent, a binder, a binder reinforcement, an emulsion stabilizer, a normal saline solution, etc. Examples of the excipient to be used include starch, dextrin, maltose, trehalose, lactose, D-glucose, sorbitol, D-mannitol, saccharose, and glycerol. Examples of the buffering agent to be used include phosphates, citrates, and acetates. Examples of the stabilizer to be used include propylene glycol and ascorbic acid. Examples of the pH adjuster to be used include: organic acids such as itaconic acid, succinic acid, tartaric acid, fumaric acid, citric acid, malic acid, adipic acid, gluconic acid, pyrophosphoric acid, acetic acid, lactic acid, α-ketoglutaric acid, and phytic acid and salts thereof; inorganic acids such as carbonic acid and salts thereof; acidic amino acids such as aspartic acid and glutamic acid; and basic amino acids such as arginine, lysine, and histidine. Examples of the preservative to be used include phenol, benzalkonium chloride, benzyl alcohol, chlorobutanol, and methyl paraben. Examples of the antiseptic to be used include ethanol, benzalkonium chloride, para-oxybenzoic acid, and chlorobutanol. Examples of the perfume to be used include: animal perfumes such as musk, civet, castoreum, and ambergris; vegetable perfumes such as anise essential oil, angelica essential oil, ylang-ylang essential oil, orris essential oil, fennel essential oil, orange essential oil, cananga essential oil, caraway essential oil, cardamom essential oil, guaiacwood essential oil, cumin essential oil, lindera essential oil, cinnamon essential oil, cinnamon essential oil, geranium essential oil, copaiba balsam essential oil, coriander essential oil, perilla essential oil, cedarwood essential oil, citronella essential oil, jasmine essential oil, ginger-grass essential oil, Japanese cedar essential oil, spearmint essential oil, peppermint essential oil, star anise essential oil, tuberose essential oil, clove essential oil, neroli essential oil, gaultheria essential oil, tolu balsam essential oil, patchouli essential oil, rose essential oil, palmarosa essential oil, cypress essential oil, cedar essential oil, sandalwood essential oil, petitgrain essential oil, bay essential oil, vetiver essential oil, bergamot essential oil, Peru balsam essential oil, bois de rose essential oil, Ho-leaf essential oil, mandarin essential oil, eucalyptus essential oil, lime essential oil, lavender essential oil, linaloe essential oil, lemongrass essential oil, lemon essential oil, rosemary essential oil, and Japanese peppermint essential oil; and other synthetic perfumes. Examples of the thickener to be used include natural polymers and starch- or cellulose-based natural polymer derivatives. Examples of the natural polymers include: seaweed extracts such as fucoidan and carrageenan; seed mucilages such as guar gum; resinous sticky substances such as gum arabic; and sticky substances produced by microorganisms such as xanthan gum. Examples of the starch- or cellulose-based natural polymer derivatives include starch-based natural polymer derivatives such as starch phosphate and cellulose-based natural polymer derivatives such as methylcellulose. Examples of the fat and oil to be used include avocado oil, linseed oil, almond oil, fennel oil, perilla oil, olive oil, orange oil, orange roughy oil, cacao butter, camomile oil, carrot oil, cucumber oil, coconut oil, sesame oil, rice oil, safflower oil, shea butter, liquid shea butter, soybean oil, camellia oil, corn oil, rapeseed oil, persic oil, castor oil, sunflower oil, grape seed oil, cottonseed oil, peanut oil, turtle oil, mink oil, egg-yolk oil, palm oil, palm kernel oil, Japan wax, coconut oil, beef tallow, and lard. Further, modified fats and oils obtained by subjecting these fats and oils to treatment such as hydrogenation, fractionation, or ester exchange may also be used. Examples of the gloss agent to be used include: waxes (including both animal and vegetable waxes) such as beeswax, carnauba wax, spermaceti wax, lanolin, liquid lanolin, hydrogenated lanolin, hard lanolin, candelilla wax, montan wax, shellac wax, rice wax, squalene, squalane, and pristane; and mineral oils such as liquid paraffin, vaseline, paraffin, ozocerite, ceresin, and microcrystalline wax. Examples of the binder to be used include soybean protein, egg protein, milk protein, blood protein, casein, starch, and transglutaminase. Examples of the binder reinforcement to be used include polyphosphates. An example of the emulsion stabilizer to be used includes casein sodium.

An embodiment of the acetaldehyde-reducing agent according to the present invention includes crushed bacterial cells of a microorganism that produces the enzyme according to the present invention (aldehyde dehydrogenase or threonine aldolase). That is, the acetaldehyde-reducing agent according to this embodiment includes crushed bacterial cells of a certain microorganism. A crushed bacterial cell solution (which is usually obtained through a series of processes of cultivation of a microorganism, collection of cells of the microorganism, and crushing of the cells) can be directly used as crushed bacterial cells. On the other hand, the cell homogenate may further be subjected to treatment (e.g., purification, freezing, drying, or addition of another ingredient) before used as crushed bacterial cells.

### 2. Use of Acetaldehyde-Reducing Agent

A second aspect of the present invention relates to a use of the acetaldehyde-reducing agent according to the present invention. The acetaldehyde-reducing agent according to the present invention can act even at low substrate concentrations. Therefore, the acetaldehyde-reducing agent according to the present invention is suitable for use in the oral cavity in which a substrate (i.e., acetaldehyde) is present at low concentrations. The present invention provides an oral composition for oral care which contains the acetaldehyde-reducing agent according to the present invention as an active ingredient. In this description, the term "oral care" refers to improving the environment in the oral cavity. The action of the oral composition according to the present invention on saliva makes it possible to reduce acetaldehyde in saliva. This "acetaldehyde-reducing" effect improves the environment in the oral cavity, which makes it possible to, for example, reduce oral odor or prevent upper digestive system cancers (e.g., pharynx cancer, larynx cancer, esophageal cancer, stomach cancer, duodenal cancer) whose cause or one of causes is acetaldehyde.

The form of the oral composition is not particularly limited as long as the oral composition can be used to act on saliva. Examples of the form of the oral composition include gums, candies, nutritional supplementary foods (e.g., supplements, energy drinks), and mouthwashes (e.g., powder dentifrice, wet dentifrice, paste dentifrice, liquid dentifrice). The oral composition according to the present invention may be prepared in the form of common foods (e.g., grain, vegetables, meat, various processed foods, confectionary, milk, soft drinks, alcohol drinks) or food additives containing the acetaldehyde-reducing agent according to the present invention. Further, the oral composition according to the present invention may be provided as a drug for preventing carcinogenesis. The form of such a drug is not particularly limited, either. Examples of the form of the drug include tablets, powders, fine granules, granules, and syrups.

The oral composition according to the present invention contains the active ingredient in an amount necessary to obtain a desired effect. The amount of the active ingredient contained in the oral composition according to the present invention may be appropriately set in consideration of the form/shape, target subject, use frequency, etc. of the oral composition. For example, the amount of the active ingredient is set to a value in the range of, for example, about 0.1 wt% to 95 wt% so that a desired amount of the active ingredient is administered (applied).

Usually, the oral composition contains, in addition to the acetaldehyde-reducing agent as an active ingredient, various ingredients depending on its form. For example, a paste dentifrice as a typical form of the oral composition may contain a thickener (moisturizing agent), a binder, a surfactant, a sweetener, a perfume, an antiseptic, a polishing agent, a pH adjuster, a chelator, a fluoride, an enzyme etc., and one or more of them may be appropriately selected, if necessary. Examples of the thickener include sorbit, glycerol, ethylene glycol, propylene glycol, 1,3-butylene glycol, polyethylene glycol, polypropylene glycol, xylit, maltit, and lactit. Examples of the binder include: synthetic binders such as carboxymethyl cellulose, sodium carboxymethyl cellulose, methylcellulose, hydroxy methylcellulose, sodium carboxymethyl hydroxymethyl cellulose, polyvinyl alcohol, and polyvinylpyrrolidone; natural binders such as xanthan gum, carrageenan, and alginic acid; and inorganic binders such as gelable silica, gelled aluminum silica, Veegum, and Laponite. As the surfactant, any of an anionic surfactant, a cationic surfactant, a nonionic surfactant, and an amphoteric surfactant may be used. Examples of the anionic surfactant include sodium lauryl sulfate, sodium N-lauroylsarcosinate, sodium dodecylbenzene sulfonate, αsodium salts-sulfofatty acid alkyl ester, sodium myristyl sulfate, sodium N-myristoyl sarcosinate, sodium hydrogenated coconut fatty acid monoglyceride monosulfate, sodium lauryl sulfoacetate, sodium α-olefin sulfonates, N-acyl glutamates such as sodium N-lauroyl glutamate and sodium N-palmitoyl glutamate, and N-acyl taurates such as sodium N-methyl-N-acyl taurate. Examples of the nonionic surfactant include sucrose fatty acid esters, polyoxyethylene fatty acid esters such as polyoxyethylene hydrogenated castor oil, polyglycerol fatty acid esters, alkyl glycosides, sorbitan fatty acid esters, polyoxyethylene sorbitan fatty acid esters such as polyoxyethylene sorbitan monostearate, alkyldimethylamine oxides, sugar-alcohol fatty acid esters such as maltitol fatty acid esters and lactol fatty acid esters, alkylol amides, fatty acid ethanol amides such as lauric acid mono- or di-ethanol amide, polyoxyethylene higher alcohol ethers, polyoxyethylene-polyoxypropylene copolymers, and polyoxyethylene polyoxypropylene fatty acid esters, and Pluronic. Examples of the amphoteric surfactant include acetic acid betaine-type surfactants such as alkyldimethylaminoacetic acid betaine and fatty acid amide propyldimethylaminoacetic acid betaine, imidazoline-type surfactants such as N-fatty acid acyl-N-carboxymethyl-N-hydroxyethylethylenediamine salts, 2-alkyl-N-carboxymethyl-N-hydroxyethylimidazolium betaine, N-alkyldiaminoethylglycines such as N-lauryldiaminoethylglycine and N-myristyldiaminoethylglycine, and sodium N-alkyl-1-hydroxyethylimidazoline betaine. Examples of the sweetener include saccharin sodium, stevioside, neohesperidin dihydrochalcone, glycyrrhizin, perillartine, thaumatin, aspartylphenylalanine methyl ester, p-methoxycinnamic aldehyde, sucrose, fructose, sodium cyclamate, stevia extract, and thaumatin. Examples of the perfume include spearmint oil, peppermint oil, wintergreen oil, sassafras oil, clove oil, eucalyptus oil, sage oil, majorana oil, thyme oil, lemon oil, orange oil, rosemary oil, cinnamon oil, pimento oil, cinnamon leaf oil, perilla oil, gaultheria oil, 1-menthol, carvone, anethole, methyl salicylate, eugenol, limonene, n-decyl alcohol, citronellol, α-terpineol, citronellyl acetate, cineol, linalool, ethyl linalool, vanillin, and thymol. Examples of the polishing agent include: inorganic materials such as crystalline silica, amorphous silica, other silica-based polishing agents, aluminosilicate, alumina, aluminum hydroxide, insoluble sodium metaphosphate, insoluble potassium metaphosphate, titanium oxide, dibasic calcium phosphate dihydrate, heavy calcium carbonate, and light calcium carbonate; and resins such as polyolefin-based resins (e.g., polyethylene, polypropylene, polybutene), halogen-containing resins (e.g., polyvinyl chloride polyvinylidene chloride), acrylic resins (e.g., methacrylic resins), resins obtained by formalization of polyvinyl acetate or a saponified product thereof, polyamide resins (e.g., nylon), styrene resin, EVA resin, silicone resin, natural rubber, polyisoprene rubber, silicon rubber, and cellulose. Examples of the pH adjuster to be used include: organic acids such as itaconic acid, succinic acid, tartaric acid, fumaric acid, citric acid, malic acid, adipic acid, gluconic acid, pyrophosphoric acid, acetic acid, lactic acid, α-ketoglutaric acid, and phytic acid and salts thereof; inorganic acids such as carbonic acid and salts thereof; acidic amino acids such as aspartic acid and glutamic acid; and basic amino acids such as arginine, lysine, and histidine. Examples of the chelator to be used include sodium pyrophosphate, sodium tripolyphosphate, and EDTA. Examples of a disinfectant to be used include triclosan, cetylpyridinium chloride, benzalkonium chloride, and benzethonium chloride. Examples of the enzyme to be used in combination include dextronase, amylase, protease, mutanase, lysozyme, lytic enzyme, and lytic enzyme. Examples of the fluoride include sodium fluoride and stannous fluoride. Another active or medicinal ingredient may be contained, and examples thereof include chlorohexidine salts, hydrocholesterol, glycyrrhetinates, glycyrrhetinic acid, chloropycae, caropeptide, vitamins, azulene, lysozyme chloride, tartar control agents, antiplaque agents, and potassium nitrate. It is to be noted that the amounts of these ingredients to be used may be appropriately set.

The target subject to which the oral composition according to the present invention is applied is not particularly limited. Examples of the target subject include humans and mammals other than humans (Pet animals, livestock animals, and laboratory animals are included. More specifically, for example, monkeys, mice, rats, guinea pigs, hamsters, monkeys, cows, pigs, goats, sheep, horses, chickens, sheep, whales, dolphins, dogs, and cats are included.).

One embodiment of the oral composition according to the present invention contains, as one of active ingredients, an acetaldehyde-degrading enzyme other than the enzyme used in the acetaldehyde-reducing agent according to the present invention. More specifically, according to this embodiment, another acetaldehyde-degrading enzyme is used in addition to the aldehyde dehydrogenase or the threonine aldolase to produce an added effect or a synergistic effect. Examples of the "another acetaldehyde-degrading enzyme" include benzoylformate decarboxylase, rhamnulose-1-phosphate aldolase, deoxyribose-phosphate aldolase, butanal dehydrogenase, carboxylate reductase, mandelonitrile lyase, 1,5-anhydro-D-fructose reductase, 3-methylbutanal reductase, indole-3-acetaldehyde reductase, gluconate 2-dehydrogenase, glycerol dehydrogenase, glyoxylate reductase, carbonyl reductase, aryl-alcohol reductase, methylglyoxal reductase, alcohol dehydrogenase, aldehyde reductase, acetoin dehydrogenase, glyceraldehyde-3-phosphate reductase, malonate-semialdehyde dehydrogenase, aminobutyraldehyde dehydrogenase, lactaldehyde dehydrogenase, L-aminoadipate-semialdehyde dehydrogenase, 4-trimethylammoniobutyraldehyde dehydrogenase, aminomuconate-semialdehyde dehydrogenase, retinal dehydrogenase, glutaminate-5-semialdehyde dehydrogenase, betaine-aldehyde dehydrogenase, 1-pyrroline-5-carboxylate dehydrogenase, fluoroacetaldehyde dehydrogenase, succinate-semialdehyde dehydrogenase, formaldehyde dehydrogenase, glucoaldehyde dehydrogenase, phenylacetaldehyde dehydrogenase, methylmalonate-semialdehyde dehydrogenase, xanthine oxidase, pyruvate oxidase, indole-3-acetaldehyde oxidase, formaldehyde dismutase, 2-hydroxy-3-oxoadipate synthase, formaldehyde transketorase, oxyaminotransferase, aldehyde ferredoxin oxidoreductase, lactate aldolase, pyruvate decarboxylase, phenylpyruvate decarboxylase, benzoin aldolase, pyruvate dehydrogenase, quinoprotein formaldehyde dehydrogenase, and 2,5-dioxovalerate dehydrogenase.
Hereinbelow, the results of experiments on reduction of acetaldehyde performed using acetaldehyde-degrading enzymes are shown.

### EXAMPLES

### (Artificial Saliva)

Artificial saliva containing 0.51% w/v NaHCO₃, 0.137% w/v K₂HPO₄ 0.088% w/v NaCl, 0.048% w/v KCl, and 0.044% w/v CaCl₂ · 2H₂O (see JP 2007-236233 A) was used. In the experiments, a two-fold concentration of artificial saliva (2×saliva sol.) was prepared and used.

### (Detection of Acetaldehyde by Nash's Method)

A solution containing acetaldehyde (200 µL) was mixed with an equal amount of Nash's reagent (15% w/v ammonium acetate, 0.5% v/v acetic acid, 2% v/v acetylacetone) and subjected to reaction at 55°C for 30 minutes, and was then subjected to measurement of absorbance at 388 nm. The initial concentration of acetaldehyde was 10 mM.

### (Detection of Acetaldehyde by GC-MS)

Since acetaldehyde is a substance having very high reactivity, detection of acetaldehyde was performed after acetaldehyde was converted into a stable derivative. A solution containing acetaldehyde (800 µL) was mixed with 160 µL of 10 mg/mL pentafluorobenzylhydroxylamine (hereinafter, referred to as "PFBOA") and 160 µL of 10% trichloroacetic acid (TCA) and reacted on ice for 30 minutes, and was then reacted at room temperature for 1.5 hours or longer. Then, 10 µL of 4-bromofluorobenzene (1 mg/ml methanol solution) was added thereto as an internal standard to obtain a sample, and 1 mL of the sample was transferred into a GC-MS vial. As a column, HP-INNOWax (Agilent Technologies) was used. It is to be noted that the oven temperature and the loop temperature for GC-MS measurement were set to 60°C and 200°C, respectively.

### 1. Measurement of Aldehyde-Reducing Activity 1 (Nash's Method)

The following three samples were prepared to compare an acetaldehyde-reducing effect. As a control, bovine serum albumin (BSA) was used, and a mixture of 0.2 mM Tris buffer (pH 8.0), 1 × saliva sol., 10 mM acetaldehyde, and 50 µg/µL BSA was prepared. As a sample for an aldehyde dehydrogenase (derived from Sacchromyces cerevisiae, A6228, Sigma-Aldrich; hereinafter, referred to as "AD"), a mixture of 20 mM Tris buffer (pH 8.0), 1 × saliva sol., 10 mM acetaldehyde, 50 µg/µL AD, and 2.5 mM NAD⁺ was used. As a sample for a threonine aldolase (derived from Escherichia coli, Eur. J. Biochem, 255, 220-226 (1998); hereinafter, referred to as "TA"), a mixture of 20 mM sodium phosphate buffer (pH 7.0), 1 x saliva sol., 10 mM acetaldehyde, 50 µg/µL TA, and 100 mM glycine was used. The amount of a reaction solution was set to 200 µL, and the reaction solution was subjected to reaction at 37°C for 30 minutes. The results are shown in Fig. 1. Both the threonine aldolase and the aldehyde dehydrogenase exhibited acetaldehyde-reducing activity. It is to be noted that the concentration of acetaldehyde was calculated based on a prepared calibration curve.

### 2. Measurement of Aldehyde-Reducing Activity 2 (GC-MS)

As in the case of measurement by Nash's method, the following three samples were prepared to compare an acetaldehyde-reducing effect.
(1) BSA sample: 18.8 µg/mL BSA, 1 × saliva sol., 100 µM acetaldehyde
(2) AD sample:
   20 mM Tris buffer (pH 8.0), 18.8 µg/mL AD, 1 × saliva sol., 100 µM acetaldehyde, 1 mM NAD⁺
(3) TA sample: 2.5 mM sodium phosphate buffer (pH 7.0), 1 × saliva sol., 100 µM acetaldehyde, 18.8 µg/µL TA, 100 mM glycine

The amount of a reaction solution was set to 800 µL, and the reaction solution was subjected to reaction at 37°C. As shown in Fig. 2, the threonine aldolase and the aldehyde dehydrogenase had a strong acetaldehyde-reducing effect. It is said that acetaldehyde exhibits oncogenic potential even at a concentration as low as 50 µM (see, for example, US2008/0166394 A1 for details). However, these two enzymes could reduce the concentration of acetaldehyde in the artificial saliva from 100 µM to a level lower than the carcinogenic concentration of acetaldehyde within 5 minutes. It is to be noted that the concentration of acetaldehyde was calculated based on a prepared calibration curve.

As has been described above, it has been found that the aldehyde dehydrogenase derived from Saccharomyces cerevisiae and the threonine aldolase derived from Escherichia coli well act on acetaldehyde present in saliva at low concentrations. Particularly, the former is very excellent in terms of the activity and rate of degradation of low concentrations of acetaldehyde (Fig. 2).

### INDUSTRIAL APPLICABILITY

The acetaldehyde-reducing agent according to the present invention can degrade acetaldehyde present in minute amounts in saliva. That is, the acetaldehyde-reducing agent according to the present invention is effective in reducing acetaldehyde in the oral cavity. The acetaldehyde-reducing agent according to the present invention can be applied to the fields of oral care, medicine, and food.

The present invention is not limited to the description of the above embodiments and examples. The present invention includes various modifications that can be easily conceived by those skilled in the art without departing from the claims.
The entire contents of literatures, patent application publications, and patent publications cited in this description are incorporated herein by reference.

## Claims

1. An agent for reducing acetaldehyde in an oral cavity, comprising an aldehyde dehydrogenase or a threonine aldolase.

2. The agent for reducing acetaldehyde in an oral cavity according to claim 1, wherein the aldehyde dehydrogenase is derived from a microorganism belonging to the genus Saccharomyces.

3. The agent for reducing acetaldehyde in an oral cavity according to claim 2, wherein the microorganism belonging to the genus Saccharomyces is Saccharomyces cerevisiae.

4. The agent for reducing acetaldehyde in an oral cavity according to claim 1, wherein the threonine aldolase is derived from Escherichia coli.

5. The agent for reducing acetaldehyde in an oral cavity according to any one of claims 1 to 4, which exhibits degradation activity against acetaldehyde at a substrate concentration of 1 µM to 1 mM.

6. The agent for reducing acetaldehyde in an oral cavity according to any one of claims 1 to 4, which exhibits degradation activity against acetaldehyde at a substrate concentration of 1 µM to 100 µM.

7. An oral composition comprising the agent for reducing acetaldehyde in an oral cavity according to any one of claims 1 to 6.

8. A method for reducing acetaldehyde in saliva, comprising allowing the oral composition according to claim 7 to act on saliva.

9. The method according to claim 8, which is effective in reducing oral odor.

10. The method according to claim 8, which is effective in preventing cancer whose cause or one of causes is acetaldehyde.
